# EUROPÄISCHE PATENTSCHRIFT

(11) **EP 2 209 516 B1**
(45) Veröffentlichungstag und Bekanntmachung des Hinweises auf die Patenterteilung: **22.05.2013**
(21) Anmeldenummer: 08848115.5
(22) Anmeldetag: 28.10.2008
(51) Int. Cl.: A61M 25/06

(54) **MEDIZINISCHES GERÄT IN FORM EINES KATHETERS ZUM ZU-, ABFÜHREN VON FLUID IN, AUS KÖRPERHÖHLEN**
MEDICAL DEVICE IN THE FORM OF A CATHETER FOR SUPPLYING FLUID TO, REMOVING FLUID FROM BODY CAVITIES
APPAREIL MÉDICAL SOUS FORME DE CATHÉTER CONÇU POUR ADMINISTRER OU ÉVACUER UN FLUIDE DANS DES CAVITÉS CORPORELLES

(30) Priorität: 06.11.2007 AT 17842007; 05.03.2008 AT 3612008
(43) Veröffentlichungstag der Anmeldung: 28.07.2010
(73) Patentinhaber: Allomed Medizintechnik GmbH, 2320 Schwechat (AT)
(72) Erfinder: MILACEK, Walter, A-1020 Wien (AT); SABEFF, Christian, A-1210 Wien (AT)
(74) Vertreter: Wagner, Matthias
(86) Internationale Anmeldenummer: PCT/AT2008/000391
(87) Internationale Veröffentlichungsnummer: WO 2009/059341

(56) Entgegenhaltungen:
- WO-A-96/40360
- GB-A- 2 065 479
- GB-A- 2 185 689
- GB-A- 2 338 898
- US-A- 4 051 852
- US-A1- 2002 193 752

## Beschreibung

Die vorliegende Erfindung betrifft ein medizinisches Gerät in Form eines Katheters zum Zu-, insbesondere jedoch Abführen von Fluid in, insbesondere aus Körperhöhlen, insbesondere dem Pleuraraum, welches Gerät einen Katheterkopf und einen Katheterschaft aufweist, wobei der Katheterkopf am proximalen Ende einen mit einem oder mehreren Dichtungs- und/oder Absperrorganen versehenen Einlass zum Einführen einer Hohl- oder Verresnadel in den Katheter aufweist, und wobei im Bereich zwischen dem (den) Dichtungs- bzw. Absperrorgan(en) und dem zum Einführen in den Körper vorgesehenen Bereich des Katheterschafts ein Abzweigeanschluss vorgesehen ist, welcher vorzugsweise über einen Abzweigeschlauch mit einer Saugpumpanordnung verbunden ist.

Das erfindungsgemäße Gerät kann verwendet werden, um Luft oder Körperflüssigkeiten mittels einer Punktion aus dem menschlichen Körper zu entfernen, es kann jedoch in gleicher Weise im veterinären Bereich Verwendung finden, wobei die vorliegende Beschreibung insbesondere auf die Verwendung in der Humanmedizin eingeht. Eine bevorzugte Verwendungsmöglichkeit ergibt sich bei der Behandlung von krankhaften Flüssigkeits- oder Gasansammlungen im Brustfellraum (Pleurahöhle).

Das Lungengewebe wird vom Lungenfell wie ein Mantel umgeben. Gleichzeitig bekleidet das Rippenfell die Innenseite der Rippen. Als Pleuraraum wird der Bereich zwischen dem Lungen- und dem Rippenfell bezeichnet, welcher bei gesunden Menschen luft- und flüssigkeitsleer ist, wobei in diesem Spalt normalerweise ein Unterdruck herrscht.

Pleuraergüsse entstehen, wenn vermehrt Pleuraflüssigkeit produziert wird, bzw. wenn der lymphatische Abtransport vermindert ist, wobei die Ursachen vielfältig sein können. Eine häufige Ursache sind bösartige Tumore, es können jedoch auch Verletzungen, Entzündungen oder eine Herzschwäche zu Pleuraergüssen führen. Mittels Ultraschalluntersuchung sind bereits Flüssigkeitsansammlungen von 20 bis 30 Milliliter nachweisbar, allerdings befinden sich, da Pleuraergüsse allmählich entstehen und die Symptome meist erst spät auftreten, zum Zeitpunkt der Diagnose oft schon einige Liter Flüssigkeit im Pleuraraum, wobei anfänglich oft nicht klar ist, aus welcher Flüssigkeit der Erguss besteht (blutig, eitrig, klar, etc.). Darüber kann durch eine diagnostische Pleurapunktion Klarheit gewonnen werden, wobei die gewonnene Flüssigkeit chemisch, zytologisch und bakteriologisch untersucht werden kann.

Die Behandlung von Pleuraergüssen hängt wesentlich von der Grunderkrankung ab, wobei bei großen Ergussmengen eine Entlastungspunktion sinnvoll sein kann, um die angesammelte Flüssigkeitsmenge zu verringern. Um den Kreislauf nicht zu belasten, und um die Gefahr des Auftretens eines Lungenödems zu verringern, wird dabei im Allgemeinen nicht die gesamte Flüssigkeitsmenge auf ein Mal entnommen, sondern meist zwischen 100 und maximal 1500 ml. Um Entzündungen vorzubeugen oder um diese zu behandeln können auch Medikamente, beispielsweise Antibiotika, über die Punktionsstelle in den Pleuraraum eingebracht werden. Bei eitrigem Erguss im Brustfellraum (Pleuraempyem) kann auch eine Spül-Saug-Drainage in den Pleuraraum erforderlich sein, bei welcher verfestigte Stoffe im Pleuraraum vor und zwischen den Absaugschritten durch Einbringen einer Spülflüssigkeit gelöst werden.

Die Pleurapunktion wird meist von einem erfahrenen Arzt unter örtlicher Betäubung des Patienten durchgeführt, indem eine Nadel (bzw. ein Punktionskatheter), gegebenenfalls unter Ultraschallkontrolle, in den Raum zwischen Rippenfell und Lungenfell (Pleuraspalt) eingeführt wird. Die Punktion kann einerseits der Gewinnung von Untersuchungsmaterial dienen (diagnostische Anwendung) andererseits können die Flüssigkeitsansammlungen abgesaugt werden (therapeutische Anwendung). Meist wird eine Kanüle oder eine von einem Kunststoffröhrchen umhüllte Hohlnadel verwendet, die zwischen zwei Rippen durch die Haut und die Zwischenrippen-Muskulatur in die Pleurahöhle eingeführt wird. Durch eine auf die Kanüle aufgesetzte Spritze kann etwas Pleuraerguss abgesaugt und zur weiteren Untersuchung in ein Labor gebracht werden. Danach wird die Hohlnadel herausgezogen und an das verbleibende Kunststoffröhrchen wird ein Schlauch angeschlossen, durch den der Erguss abgeleitet wird. Als Verbindungselemente werden meist sogenannte Luer-Verbindungen oder Luer-Locks verwendet, da diese international verwendet werden, und eine einheitlich genormte (etwa in der DIN 13090, der EN 1707 oder der ISO 594/1) Verbindung unterschiedlicher medizinischer Geräte ermöglichen.

Das Absaugen kann entweder über eine Spritze erfolgen, oder durch spezielle Unterdruck- oder Pumpsysteme, die an den Katheter angeschlossen werden und insbesondere dem Abführen größerer Flüssigkeits- oder Gasmengen dienen. Zum Abpumpen von Flüssigkeit wird üblicherweise eine einfache Absaugvorrichtung verwendet, die im Wesentlichen aus einem verstellbaren Dreiwegeventil besteht, wobei an einen Ast des Ventils, meist über einen Luer-Lock, eine Spritze angeschlossen ist, der andere Ast zu einem Sammelbeutel für das abgesaugte Sekret führt, und der dritte Ast über den Pleurapunktionskatheter die Verbindung mit dem Pleuraraum herstellt. Zum Absaugen wird zuerst das Ventil mit einem Handhebel so eingestellt, dass die Verbindung zum Sammelbeutel getrennt ist, und durch Aufziehen der Spritze die Flüssigkeit aus dem Pleuraraum in die Spritze abgesaugt wird. Ist die Spritze gefüllt, wird der Ventilhebel umgeschaltet, sodass die Verbindung zum Katheter verschlossen ist, und die abgesaugte Flüssigkeit wird nun durch Zusammendrücken der Spritze in den Sammelbeutel gedrückt, der nun über das Ventil mit der Spritze in Verbindung steht. Diese Anordnung erfordert ein ständiges händisches Umschalten des Ventils, wobei ein Fehler beim Umschalten dazu führen könnte, dass die Flüssigkeit aus dem Beutel abgesaugt, bzw. gar in den Pleuraraum zurückgespritzt würde.

Nach dem Absaugvorgang können über den Katheter gegebenenfalls Medikamente in den Pleuraraum eingebracht werden, beispielsweise ein örtliches Betäubungsmittel, ein Antibiotikum, ein Gewebekleber, ein zellabtötendes Mittel (Zytostatikum) oder schmerzstillende Medikamente. Zuletzt wird das Katheter-Kunststoffröhrchen herausgezogen und ein Verband angelegt.

Bei herkömmlichen Kathetern muss zwischen einzelnen Arbeitsschritte oft eine andere Vorrichtung an den Katheter angeschlossen werden, wobei immer sichergestellt sein muss, dass keine Luft in den Pleuraraum eindringen kann, da dies schnell zu einem Pneumothorax (Gasbrust) führen könnte. Dazu dienen verschiedene Sperrventile, deren richtige Bedienung in der Verantwortung des Arztes liegt. Neben der Bedienung der Einrichtungen muss der Arzt darüber hinaus dem Patienten Anweisungen erteilen, etwa wann er die Luft anhalten, bzw. aus- oder einatmen soll. Fehler werden somit ausschließlich durch die fachliche Kompetenz und die Umsicht des Arztes vermieden, da die verwendeten Vorrichtungen Fehlbedienungen nicht verhindern.

Als mögliche Komplikationen beim Setzen eines Pleurakatheters können Infektionen auftreten, dies ist jedoch bei steriler Nadel und guter Hautdesinfektion selten. Zudem ist es möglich, dass es beim Einführen des Katheters, oder wenn der Patient sich bewegt oder hustet, zu einer Verletzung der Lunge kommt. Auch Verletzungen von Leber oder Milz sowie Nachblutungen können auftreten.

Um Verletzungen der Lunge und der inneren Organe zu verhindern, können beispielsweise beim Punktieren spezielle, als "Verresnadeln" bezeichnete Nadeln verwendet werden, die im Fachgebiet bereits seit langem bekannt sind. Verresnadeln erlauben das Durchstechen von hartem Material (Muskelgewebe, Rippenfell), verhindern aber ein Verletzten von weichem Material (Lungenfell, Lungengewebe). Zu diesem Zweck besteht eine Verresnadel aus einer Außennadel mit einem scharfen distalen Ende (ähnlich einer Kanüle), in deren Lumen sich eine Innensonde erstreckt, die eine stumpfe Sondenspitze aufweist. Die Innensonde ist mittels einer Feder nach vorne hin vorgespannt, sodass ihre stumpfe Sondenspitze über die Spitze der Außennadel heraussteht. Beim Durchstechen eines verhältnismäßig festen Gewebes, wie etwa der Brustmuskulatur, wird die Sondenspitze gegen die Federkraft in die Außennadel hinein zurückgeschoben, sodass das scharfe Ende der Außennadel freiliegt, und wie eine normale Kanülenspitze in das Gewebe eindringen kann. Sobald die Nadelspitze in weicheres Gewebe gelangt, drückt sich die Sondenspitze durch die Federkraft wieder aus der Außennadel heraus, und drängt dabei das weichere Gewebe von der Spitze der Außennadel weg, sodass das Gewebe nicht beschädigt wird. Um die Verresnadel wie eine herkömmliche Kanüle verwenden zu können, ist die Sonde hohl ausgebildet, wobei die distale Öffnung nicht an der Spitze, sondern seitlich davon vorgesehen ist.

Die Verwendung einer Verresnadel in einem medizinischen Instrument für eine Pleurapunktion ist beispielsweise in der DE 693 28 254 beschrieben. Die DE 693 28 254 offenbart weiters ein Kugelventil, welches, sobald die Verresnadel aus dem Katheter herausgezogen wird, den Nadeleinlass des Pleurapunktionskatheters verschließt, um das Eindringen von Luft in den Pleuraraum zu verhindern, wobei das Ventil aus mehreren beweglichen Teilen besteht (Feder, Kugel, Kolben). Der Kopf des Katheters weist eine Abzweigung auf, von der ein Schlauch zu einer Absaugeinheit führt, die in herkömmlicher Weise ein Dreiwegeventil benutzt, wie dies oben beschrieben ist.

In zunehmenden Maße werden in jüngerer Zeit Rückschlagventile aus einem elastischen Material, wie z.B. Gummi oder einem anderen Elastomer, im medizinischen Bereich verwendet. Diese Ventile bestehen aus einem im Allgemeinen einteiligen Körper, der meist eine innen hohle, im Wesentlichen zylindrische Form aufweist, und an einer Seite keilförmig zusammenläuft, sodass an der Keilspitze der innere Hohlraum durch zwei aneinander anliegende Dichtlippen abgeschlossen ist. Diese Ventile werden im Allgemeinen als Lippenventile, oder aufgrund ihrer Form als (Enten-)Schnabelventile bezeichnet. Aufgrund ihres einfachen Aufbaus sind solche Ventile in vielen Ausbildungen verfügbar, wobei die Spitze nicht immer keilförmig sein muss, sondern bei einigen Modellen auch konisch zulaufend oder kreuzförmig (ähnlich der Form eines Kreuzschraubenziehers) ausgebildet sein kann. Lippenventile sind kostengünstig, sie passen auf kleinsten Raum, benötigen nur ein einfaches Gehäuse, sind korrosions- und verschleißbeständig, selbstreinigend sowie sicher und leicht zu montieren. Lippenventile können auch für verschiedenste Druckdifferenzen ausgelegt werden und durch Variieren der Form und des Materials an spezifische Anforderungen angepasst werden. Verschiedene Modelle von Lippenventilen für den medizinischen Bereich finden sich beispielsweise in den Produktkatalogen der Firma Vernay Laboratories, Inc., Yellow Springs, Ohio, USA.

Darüber hinaus ist es aus der W02006/103233 bekannt, ein in einer Abzweigung eines Endotrachealtubus angeordnetes Lippenventil als abdichtenden Eingangsport für einen Katheter zu verwenden.

Es ist ein Ziel der vorliegenden Erfindung, einen Pleurapunktionskatheter bereitzustellen, der ein Eindringen von Luft in den Pleuraraum zuverlässig verhindert, und dabei einfacher in der Anwendung und günstiger in der Herstellung ist, als derzeit für die Pleurapunktion verwendete medizinische Geräte. Zur Lösung dieser Aufgaben wird erfindungsgemäß ein Gerät vorgeschlagen, dessen Abzweigeschlauch zu einem Ansaugventil führt, welches den Durchfluss eines Fluids in Richtung Saugeinrichtung ermöglicht, jedoch in der Gegenrichtung den Durchfluss sperrt, wobei der Auslass des Ansaugventils in einer Gablung mündet, von der ein Ast zu einem, vorzugsweise als Luer-Lock ausgebildeten Saugeinrichtungsanschluss, insbesondere einem Spritzenanschluss führt, und deren anderer Ast zu einem Sekretbeutelanschluss führt, wobei zwischen diesem Ast und dem Sekretbeutelanschluss ein den Rückfluss vom Sekretbeutel sperrendes Rückschlagventil angeordnet ist. Pleurapunktionskatheter sind bekannt aus der GB 2185689, GB 2338898 sowie US 4051852.

Das erfindungsgemäß ausgebildete medizinische Gerät ermöglicht ein einfaches und sicheres Punktieren des Pleuraraum oder einer anderen Körperhöhlung, wobei über die gesamte Behandlungsdauer ein Eindringen von Luft oder Fremdstoffen ins Körperinnere vermieden wird. Darüber hinaus enthält das erfindungsgemäße medizinische Gerät eine einfache und effektive Absaugvorrichtung, deren Handhabung weniger aufwändig ist, als bei derzeitigen Systemen, und die sowohl zum Absaugen von Flüssigkeiten, als auch von Gasen geeignet ist. Insbesondere muss der Arzt beim Absaugen keine Ventile manuell umstellen, sodass er seine Aufmerksamkeit mehr auf die Behandlung des Patienten richten kann, anstatt auf die Bedienung der Vorrichtung. Mit dem erfindungsgemäßen Katheter ist auch ein Zuführen von Fluid in die Körperhöhle möglich, ohne dass die Kathetervorrichtung oder Teile davon ausgewechselt werden müssen. Diese Möglichkeit kann beispielsweise dazu genutzt werden, um nach dem Absaugen Medikamente in den Pleuraraum einzuspritzen, oder um die Vorrichtung als Spül-Saug-Drainage zu verwenden, wobei verfestigte Substanzen in der Körperhöhle vor dem Absaugen durch eine Spülung gelöst werden.

In einer vorteilhaften Ausführungsform kann der Sekretbeutelanschluss als Luer-Lock ausgebildet sein, wobei das den Rückfluss vom Sekretbeutel sperrende Rückschlagventil ein Schnabel- bzw. Lippenventil oder ein Membran-Rückschlagventil sein kann, welches im Inneren des Luer-Lock angeordnet ist. Auch das Ansaugventil kann als Schnabel- bzw. Lippenventil oder ein Membran-Rückschlagventil ausgebildet sein. Schnabel- oder Lippenventile (die auch unter der Bezeichnung "Duckbill-Ventil" bekannt sind) sind besonders einfache und günstige Ventile und eignen sich daher besonders für die Verwendung in Einweggeräten. Auch Membran-Rückschlagventile sind preisgünstig herzustellen und haben überdies den Vorteil sehr geringer Einbaumaße. Das Ventil im Luer-Lock des Sekretbeutelanschlusses anzuordnen stellt eine besonders günstige Ausführungsform dar, da die Anzahl unterschiedlicher funktioneller Einheiten minimiert wird.

In einer weiteren Ausführungsform kann der Abzweigeschlauch zwischen dem Katheter und dem Ansaugventil vorzugsweise mittels einer Schlauchklemme sperrbar sein. Dadurch kann die Verbindung vom Katheter zur Saugpumpenanordnung rasch und unkompliziert unterbrochen werden, sodass der Katheter zum Einleiten von Fluid in den Körper verwendet werden kann.

Die Erfindung gemäß Anspruch 1 sieht vor, dass der Einlass zum Einführen der Hohl- oder Verresnadel ein nach innen gerichtetes, als Durchstoßventil ausgebildetes Schnabel- bzw. Lippenventil aufweist, welches am Umfang der eingeschobenen Hohl- oder Verresnadel abdichtend anliegt, und bei herausgezogener Nadel den Fluiddurchlass unter den zwischen der Körperhöhle und dem Außenbereich üblicherweise auftretenden Druckunterschieden in beiden Richtungen sperrt. im Katheterkopf zwischen dem Durchstoßventil und dem Abzweigeanschluss ist ein manuell betätigbares Sperrventil angeordnet. Dadurch kann die Nadel, die dem Einsetzen des Katheters dient, einfach aus dem Katheter herausgezogen werden, sobald dieser in Position gebracht ist. Durch das Ventil wird der Nadeleinlass sofort verschlossen, sodass keine Luft ins Körperinnere eindringen kann. Ein manuell betätigbares Sperrventil dient der zusätzlichen Sicherheit und sperrt den Nadeleinlass auch bei höheren Druckunterschieden, die etwa beim Abpumpen oder beim Einatmen auftreten können. Wird das Sperrventil geöffnet, können über den Einlass etwa mit einer Spritze Flüssigkeiten über den Katheter in die Körperhöhle eingebracht werden. Dazu muss das Durchstoßventil nicht notwendigerweise mit einer Nadel durchstoßen werden, sondern es reicht etwa eine Spritze aus, die über eine Luer-Verbindung an den Katheterkopf angeschlossen ist, da das Ventil bei Druckunterschieden, die über dem Öffnungsdruck liegen, öffnet und das Einbringen von Flüssigkeiten erlaubt. Dieser Abschluss des Nadeleinlasses ist zudem technisch einfacher und erheblich günstiger herzustellen, als bekannte Systeme, welche etwa Kugelventile verwenden.

die Saugpumpenanordnung gemäß Anspruch 1 einschließlich dem Ansaugventil, der Gabelung, dem Ast zum Spritzenanschluss, dem Ast zum Sekretbeutelanschluss, dem Spritzenanschluss, dem Sekretbeutelanschluss und dem Rückschlagventil ist in einem gemeinsamen Bauteil als T-Stück ausgebildet, wobei das Ansaugventil und/oder das Rückschlagventil als Membran-Rückschlagventil ausgebildet sein kann. Ein solches T-Stück kann mit verhältnismäßig wenig Einzelteilen hergestellt werden und bietet Vorteile bei der maschinellen Fertigung, da bei der Herstellung des T-Stücks mittels Spitzguss-Verfahren einfache Spritzguss-Formen verwendet werden können die ohne Schieber auskommen. Durch die Verwendung von Membran-Rückschlagventilen als Ansaugventil bzw. Rückschlagventil lässt sich eine besonders kompakte Ausführung der Saugpumpenanordnung erzielen.

Besondere Ausführungsformen der Erfindung werden im Folgenden anhand beispielhafter Zeichnungen beschrieben. Es zeigt
Fig. 1 einen beispielhaften Katheter mit der daran angebrachten
   Saugpumpenanordnung in einer Schnittansicht,
Fig. 2 den Katheter ohne der Saugpumpenanordnung in einer Draufsicht,
Fig. 3 den Nadeleinlass des Katheterkopfes mit einer darin angeordneten Spitze einer Verresnadel und dem Sperrventil in einer Schnittansicht,
Fig. 4 einen beispielhaften Katheter mit einer am Abzweigeschlauch
   vorgesehenen Schlauchklemme,
Fig. 5 einen Teil der beispielhaften Saugpumpenanordnung, wobei im Inneren des Sekretbeutelanschlusses ein als Schnabelventil ausgebildetes Rückschlagventil angeordnet ist,
Fig. 6 eine als T-Stück ausgebildete Saugpumpeneinheit in teilweise aufgeschnittener Darstellung,
Fig. 7 das im T-Stück der Fig. 6 als Ansaugventil verwendete Membran-Rückschlagventil in einer vergrößerten Darstellung,
Fig. 8 in einer schematischen Darstellung die Funktionsweise des Membran-Rückschlagventils,
Fig. 9 eine Draufsicht auf die Ventilmembran, und
Fig. 10 die erfindungsgemäße Katheteranordnung mit dem T-Stück.

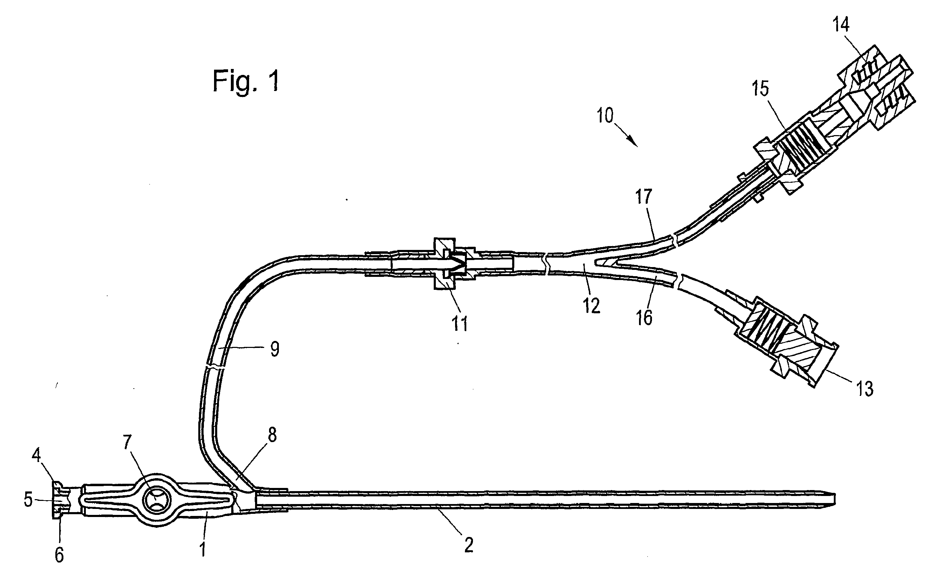

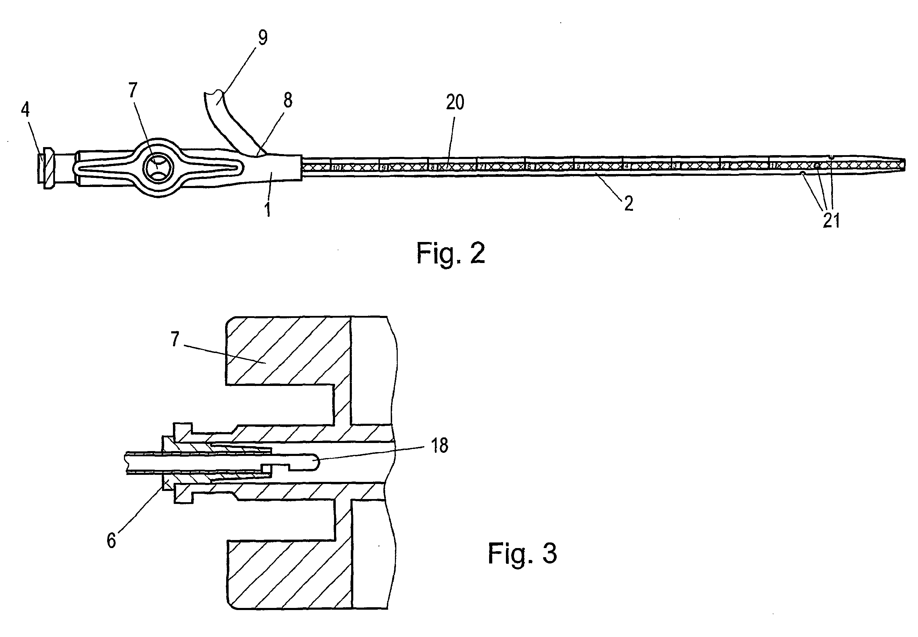

Fig. 1 zeigt einen beispielhaften Pleurapunktionskatheter 3 mit der Saugpumpenanordnung 10, die über einen Abzweigeschlauch 9, der von einem Abzweigeanschluss 8 vom Katheter 3 wegführt, mit dem Katheter 3 verbunden ist. Der Katheter 3 besteht im Wesentlichen aus einem Katheterschaft 2 und einem Katheterkopf 1, wobei am Katheterkopf ein Sperrventil 7 angebracht ist, welches in der geöffneten Position das Eüifügen einer Nadel in den Katheter ermöglicht, und in geschlossener Position den Katheterkopf abschließt. Am proximalen Ende 4 des Katheters 3 ist als Nadeleinlass 5 ein Durchstoßventil 6 angeordnet, welches in Fig. 3 in einer detaillierteren Schnittansicht genauer dargestellt ist. Die Fig. 3 zeigt die Spitze einer Verresnadel 18, welche gerade in den Nadeleinlass 5 eingeschoben wird. Dabei dichtet das Durchstoßventil 6 die Nadel an ihrem Umfang ab. Mit der vollständig in den Katheter 3 eingeführten Nadel kann der Katheterschaft 2 in bekannter Weise in die Körperhöhle, insbesondere in den Pleuraraum, eingeschoben werden, wobei die Nadelspitze dabei ein wenig über das distale Ende des Katheterschafts 2 hinaus ragt. Dieser Vorgang kann mittels bildgebender Verfahren, wie etwa durch Ultraschall oder Röntgentechniken, überwacht werden, wobei der Katheterschaft 2 einen röntgenundurchlässigen Streifen 20 aufweist (Fig. 2), an dem beispielsweise alle zehn Millimeter Markierungen vorgesehen sind, sodass die Dimensionen auf einem Röntgenbild erkannt werden können. Am distalen Ende des Katheterschafts 2 sind vier um den Umfang des Schafts spiralartig angeordnete Auslässe 21 vorgesehen, die eine Fluidkommunikation mit dem Körperhohlraum verbessern.

Nachdem der Katheter 3 gesetzt und gegebenenfalls dessen korrekte Lage geprüft worden ist, wird die Nadel aus dem Katheter herausgezogen, sodass nur der flexible Katheterschaft 2 im Körper des Patienten verbleibt. Das im Einlass 5 angeordnete Durchstoßventil 6 weist vorzugsweise einen Öffnungsdruck auf, der so hoch ist, dass das Ventil bei den zu erwartenden Druckunterschieden zwischen Körperhohlraum und Umgebung noch nicht öffnet. Dadurch verhindert das Ventil bei herausgezogener Nadel, dass Luft in den Körper eingesogen wird. Dies ist insbesondere bei einer Behandlung des Pleuraraums wichtig, da ein schlecht schließendes Ventil zum Eindringen von Luft in den Pleuraraum, und im schlimmsten Fall zu einem Spannungspneumothorax führen könnte, der eine lebensbedrohliche Komplikation darstellt. Allerdings würde ein erfahrener Arzt diese Situation wohl sofort erkennen und stoppen können.

Der zusätzlichen Betriebssicherheit der Vorrichtung dient außerdem ein Sperrventil 7, welches zwischen dem Nadeleinlass 5 und dem Abzweigeanschluss 8 am Katheterkopf 1 angeordnet ist. Vorzugsweise wird die Nadel zuerst bis zu der in Fig. 3 gezeigten Position zurückgezogen, sodass das Sperrventil 7 geschlossen werden kann, bevor die Nadel 18 ganz aus dem Nadeleinlass 5 herausgezogen wird. Mit dieser Vorgehensweise können auch Ventile mit einem geringeren Öffnungsdruck verwendet werden, ohne dass es zu einer Gefährdung des Patienten durch eindringende Luft kommen kann. Optional könnte der Nadeleinlass 5 auch mit einem einfachen Deckel verschlossen werden, sobald die Nadel entfernt ist.

Ist nun der Katheter in Position gebracht und der Nadeleinlass 5 verschlossen, kann mit dem Absaugen der Flüssigkeit oder des Gases aus dem Pleuraraum begonnen werden. Dazu dient die Saugpumpenanordnung 10, die im Wesentlichen aus einem Ansaugventil 11, einer Gabelung 12, einem Spritzenanschluss 13, einem Sekretbeutelanschluss 14, sowie den dazwischenliegenden Verbindungsschläuchen besteht. Die Saugpumpenanordnung 10 ist über einen Abzweigeschlauch 9 am Abzweigeanschluss 8 mit dem Katheter verbunden. Der Spritzenanschluss 13 ist ein handelsüblicher und international genormter Luer-Anschluss, an dem eine Spritze (nicht dargestellt) angeschlossen wird, welche als "Pumpe" für die Saugpumpenanordnung 10 dient. Beim Sekretbeutelanschluss 14 ist, ebenfalls über eine Luer-Verbindung, ein Sammelbeutel (nicht dargestellt) für das abgesaugte Sekret, oder ein anderer Sekretbehälter angeschlossen. Der Sekretbeutelanschluss 14 weist überdies noch ein Rückschlagventil 15 auf, welches verhindert, dass Sekret aus dem Sekretbeutel zurück in die Katheteranordnung gesaugt wird und damit eine Pumpwirkung ermöglicht.

Diese Anordnung erlaubt es dem Arzt, eine beliebige Menge an Fluid abzupumpen, ohne dabei ein Ventil umstellen zu müssen. Zuerst wird die leere, am Spritzenanschluss 13 angebrachte Spritze aufgezogen, und saugt das Sekret über den Katheterschaft 2, den Abzweigeanschluss 8, den Abzweigeschlauch 9 und das Ansaugventil 11 an. Das Rückschlagventil 15 ist dabei geschlossen, sodass die Spritze kein Fluid aus dem Sekretbeutel ansaugen kann. Sobald die Spritze ausreichend gefüllt ist, drückt der Arzt das abgesaugte Sekret wieder aus der Spritze heraus, wobei sich das Ansaugventil 11 schließt, sodass kein Sekret zurück in den Körperhohlraum gedrückt werden kann. Stattdessen öffnet nun das Rückschlagventil 15 des Sekretbeutelanschlusses 14, und das abgepumpte Fluid gelangt somit in den Sammelbehälter. Dieser Pumphub kann nach Bedarf beliebig oft wiederholt werden, wobei eine Kontrolle der abgepumpten Menge entweder durch Summieren des jeweils abgesaugten Spritzeninhalts, oder durch ein externes Messystem für den Beutelinhalt erfolgen kann. Optional kann die Pumpwirkung nicht durch eine Spritze, sondern beispielsweise durch einen Pumpballon bewirkt werden, wobei aus hygienischen Gründen eine Einwegspritze zu bevorzugen ist.

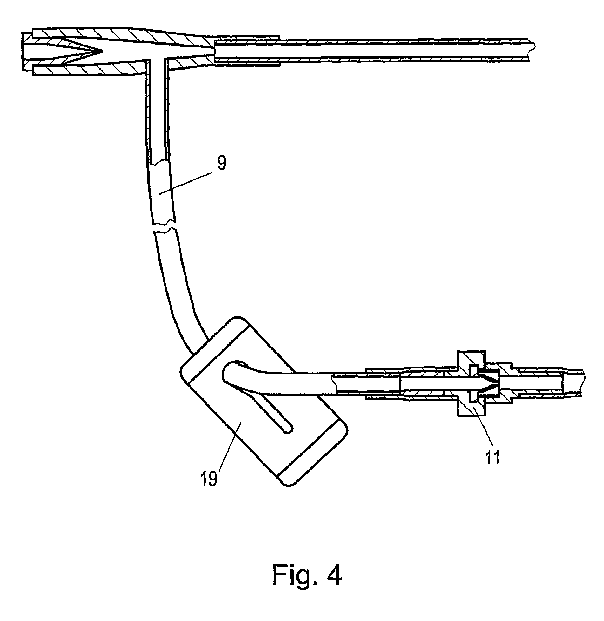

Wenn die gewünschte Fluidmenge abgepumpt worden ist, kann das System wie ein herkömmlicher Katheter entfernt werden. In manchen Fällen ist es jedoch notwendig bzw. medizinisch sinnvoll, ein Medikament in den Körperhohlraum einzubringen, bevor der Katheter entfernt wird. Auch das kann mit dem bespielhaften Katheter durchgeführt werden, ohne dass die Saugpumpenanordnung 10 zuvor entfernt werden müsste. Um zu verhindern, dass ein über den Einlass 5 eingebrachtes Mittel nicht über den Katheterschaft 2 in den Körperhohlraum gelangt, sondern stattdessen über den Abzweigeschlauch 9, das Ansaugventil 11 und das Rückschlagventil 15 zum Sekretbeutel fließt, reicht es aus, den Abzweigeschlauch 9 mittels einer einfachen Schlauchklemme zu verschließen. In Fig. 4 ist eine besonders einfache Ausführung einer solchen Schlauchklemme 19 dargestellt.

Ein Absperren des Abzweigeschlauchs 9 ist auch notwendig, wenn zwischen einzelnen Absaugvorgängen eine Spülflüssigkeit in den Pleuraraum eingebracht werden muss, etwa um verfestigte Substanzen vor dem Absaugen zu lösen. In beiden obigen Fällen kann das Medikament bzw. das Spülmittel entweder über eine Spritzennadel, die in den Nadeleinlass 5 eingesteckt wird, oder über eine andere Zuführung, die über einen Luer-Anschluss am proximalen Ende 4 des Katheters 3 dicht angeschlossen werden kann, eingebracht werden.

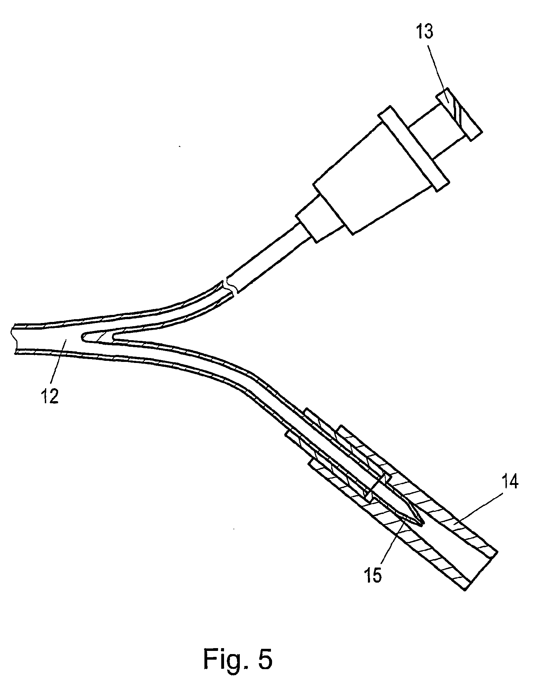

In Fig. 5 ist eine alternative Ausführungsform dargestellt, bei welcher im Luer-Lock des Sekretbeutelanschlusses 14 anstatt des in Fig. 1 dargestellten Rückschlagventils 15 ein Schnabel- bzw. Lippenventil angeordnet ist. Die Funktionsweise ist analog zu der oben dargelegten, wobei die Vorteile des preisgünstigen Schnabelventils auch beim Sekretbeutelanschluss 14 zur Geltung kommen.

Durch die hohe Flexibilität, welche die Verwendung von Lippenventilen bietet, kann die biespielhafte Vorrichtung sowohl zum Absaugen von Flüssigkeiten, als auch von Gasen aus dem Körperhohlraum verwendet werden.

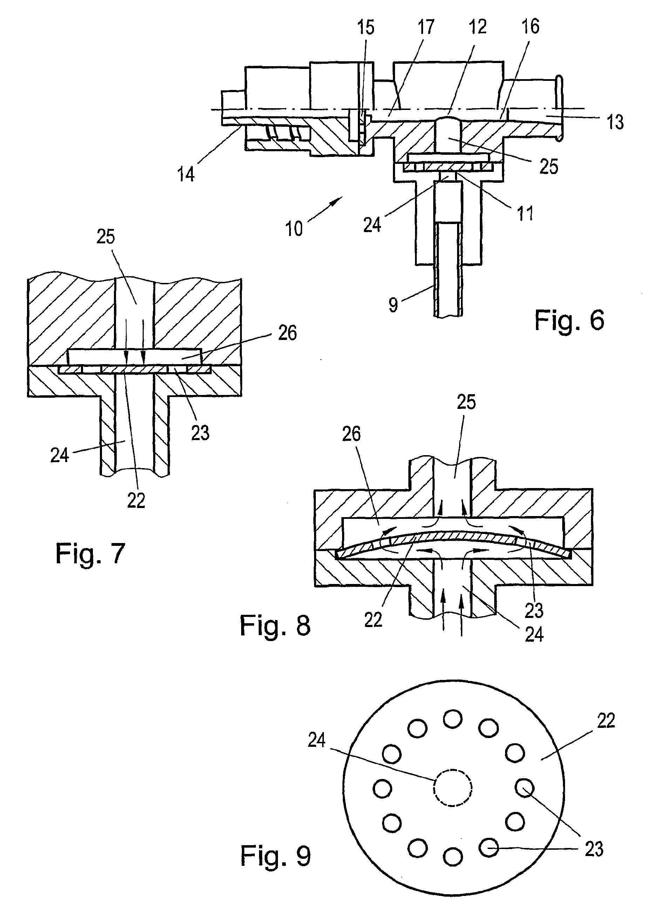

Die Saugpumpeneinheit 10 des erfindungsgemäßen Pleurapunktionskatheters ist in Fig. 6 dargestellt. Die ganze Saugpumpeneinheit 10 ist in einem einzigen sehr kompakten Bauteil integriert und als T-Stück ausgebildet. Das T-Stück ist in Fig. 6 teilweise aufgeschnitten dargestellt, um die Ventile und den Verlauf der Lumen, die Anschlüsse und die Ventile darzustellen.

Als Ventile kommen in dieser Ausführungsform Membran-Rückschlagventile zum Einsatz, die aufgrund ihrer geringen Einbaumaße eine sehr kompakte Bauweise ermöglichen. Von der Gabelung 12 ausgehend bilden in die eine Richtung der Ast 16 zum Spritzenanschluss 13 und in die andere Richtung der Ast 17 zum Sekretbeutelanschluss 14 einen Querteil mit einem im Wesentlichen gerade durchlaufenden Lumen, das nur vom Rückschlagventil 15 unterbrochen wird. Das Rückschlagventil 15 ist vor dem Sekretbeutelanschluss 14 angeordnet und lässt eine Strömung zum Anschluss 14 des Sekretbeutels zu. An der Gabelung 12 mündet der Ventilausgang 25 des Ansaugventils 11 in den Querteil.

Der Aufbau der Membran-Rückschlagventile ist in den Figuren 7 bis 9 beispielhaft dargelegt. Fig. 7 zeigt eine Schnittdarstellung des Ventils in einer gesperrten Stellung. Das Sperren des Ventils wird von einer scheibenförmigen Ventilmembran 22 bewirkt, die im Inneren einer Ventilkammer 26 so angeordnet ist, dass die Membran 22 in ihrem mittleren Bereich den Ventileingang 24, der in die Ventilkammer mündet, abdeckt. Die Ventilmembran 22 kann an ihrem Rand etwa durch Kleben oder Klemmen fix mit dem Ventilgehäuse verbunden sein, wie dies in Fig. 7 angedeutet ist, sie könnte jedoch auch in der Ventilkammer 26 schwimmend beweglich eingelegt sein. Wenn der Druck im Ventilausgang 25 ansteigt, wird die Ventilmembran 22 gegen die Mündung des Ventileingangs 24 gedrückt, und der Durchfluss somit gesperrt. Umgekehrt wird bei ansteigendem Druck in Durchflussrichtung die Membran von der Mündung des Ventileingangs 24 blasenartig abgehoben. Im radialen Randbereich der Ventilmembran 22 sind Membrandurchlässe 23 angeordnet, durch welche das Medium bei angehobener Membran fließt und in den Ventilausgang 25 gelangt. Diese geöffnete Ventilstellung ist in Fig. 8 schematisch dargestellt, Fig. 9 zeigt die Ventilmembran 22 in einer Draufsicht, wobei die radiale Anordnung der Membrandurchlässe 23 zu erkennen ist. Anstatt der Ventilmembran 22 könnte auch eine starre Scheibe verwendet werden, die schwimmend in der Ventilkammer 26 angeordnet ist, sodass sie beim Sperren auf die Mündung des Ventileingangs 24 gedrückt wird und sich beim Öffnen von dieser abhebt. Es ist bei der Auslegung der Ventilkammer und der Membran bzw. Scheibe darauf zu achten, dass die Sperrscheibe bzw. -membran in der geöffneten Ventilstellung nicht den Ventilausgang abdecken kann.

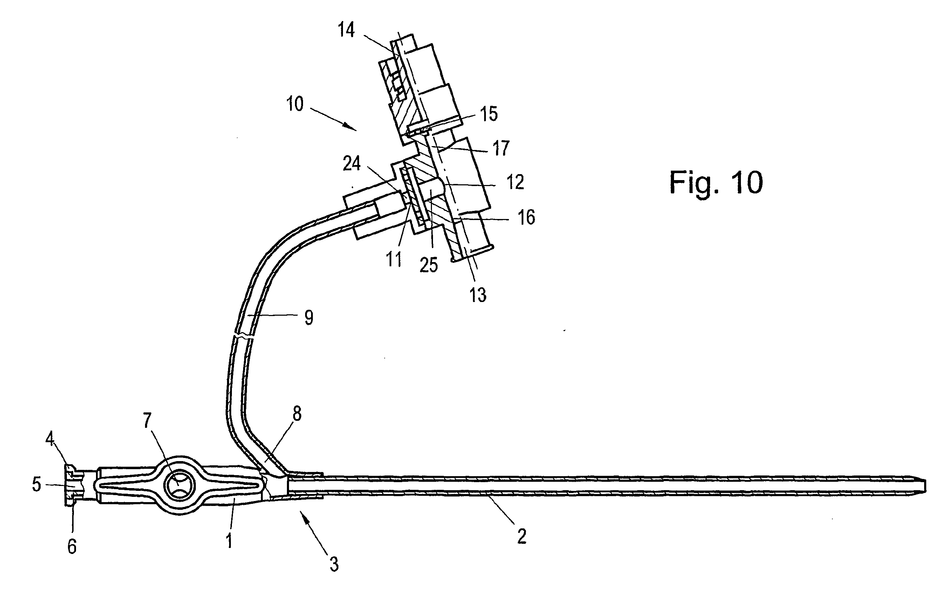

Fig. 10 zeigt den erfindungsgemäßen Pleurapunktionskatheter mit der als T-Stück ausgebildeten Saugpumpenanordnung 10. Wird über eine am Anschluss 13 angesetzte Spritze im T-Stück ein Unterdruck erzeugt, wird das aus der Körperhöhle über den Katheter abzusaugende Medium über die Leitung 9, das Ansaugventil 11 und den Ast 16 des T-Stückes in die Spritze eingesaugt. Das Rückschlagventil 15 verhindert, dass bereits im Sekretbeutel befindliches Medium zurück in die Saugpumpeneinheit 10 dringt. Danach wird mit der Spritze Druck auf das in der Spritze befindliche Medium ausgeübt, sodass dieses aus der Spritze in den Querteil des T-Stücks und über das Rückschlagventil 15 in den am Anschluss 14 angebrachten Sekretbeutel verdrängt wird. Hierbei verhindert das Ansaugventil 11, dass das Medium zurück in den Katheter und weiters gegebenenfalls auch noch in die Körperhöhle gelangt.

## Patentansprüche

1. Medizinisches Gerät in Form eines Katheters (3) zum Zu-,
Abführen von Fluid in, aus Körperhöhlen welches einen Katheterkopf (1) und einen Katheterschaft (2) aufweist, wobei der Katheterkopf (1) am proximalen Ende (4) einen mit einem selbstdichtenden Durchstoßventil (6) versehenen Einlass (5) zum Einführen einer Hohl- oder Verresnadel (18) in den Katheter (3) aufweist, und wobei im Bereich zwischen dem Durchstoßventil (6) und dem zum Einführen in den Körper vorgesehenen Bereich des Katheterschafts (2) ein Abzweigeanschluss (8) vorgesehen ist, welcher über einen Abzweigeschlauch (9) mit einer Saugpumpanordnung (10) verbunden ist, wobei die Saugpumpenanordnung (10) als T-Stück ausgebildet ist, welches ein Ansaugventil (11) in einem zum Abzweigschlauch (9) führenden Ast des T -Stückes, ein Rückschlagventil (15) in einem zum Sekretbeutelanschluss (14) führenden Ast des T-Stückes sowie einen als Spritzenanschluss (13) dienenden Ast aufweist, **dadurch gekennzeichnet, dass** ein manuell betätigtes Absperrventil (7) zwischem dem Durchstoßventil (6) des Katheterkopfs (1) und dem Abzweigeanschluss (8) angeordnet ist.

2. Gerät nach Anspruch 1, **dadurch gekennzeichnet, dass** das Ansaugventil (11) und das Rückschlagventil (15) als Membran-Rückschlagventile ausgebildet sind.

3. Gerät nach Anspruch 1 oder 2, **dadurch gekennzeichnet, dass** eine scheibenförmige Ventilmembran (22) im Inneren einer Ventilkammer (26) angeordnet ist, wobei die Ventilmembran (22) mit ihrem mittleren Bereich den Ventileingang (24) abdeckt.

4. Gerät nach einem der Ansprüche 1 bis 3, **dadurch gekennzeichnet, dass** die Ventilmembran (22) mit ihrem äußeren Rand mit dem Ventilgehäuse verbunden ist, wobei im radialen Randbereich der Ventilmembran (22) Membrandurchlässe (23) angeordnet sind.

5. Gerät nach einem der Ansprüche 1 bis 3, **dadurch gekennzeichnet, dass** die Ventilmembran (22) als starre Scheibe ausgebildet ist, die schwimmend in der Ventilkammer (26) angeordnet ist.

## Claims

1. Medical device in the form of a catheter (3) for supplying fluid to body cavities and removing it therefrom, which has a catheter head (1) and a catheter shaft (2), wherein the catheter head (1), at the proximal end (4), has an inlet (5), which is provided with a self-sealing push-through valve (6), to introduce a hollow or Veress needle (18) into the catheter (3), and wherein a branch connection (8) is provided in the region between the push-through valve (6) and the region of the catheter shaft (2) provided for introduction into the body, said branch connection being connected by a branch tube (9) to a suction pump arrangement (10), the suction pump arrangement (10) being configured as a T-piece, which has a suction valve (11) in a branch of the T-piece leading to the branch tube (9), a non-return valve (15) in a branch of the T-piece leading to the secretion bag connection (14) and a branch being used as a syringe connection (13), **characterised in that** a manually actuated check valve (7) is arranged between the push-through valve (6) of the catheter head (1) and the branch connection (8).

2. Device according to claim 1, **characterised in that** the suction valve (11) and the non-return valve (15) are configured as diaphragm non-return valves.

3. Device according to claim 1 or 2, **characterised in that** a disc-like valve diaphragm (22) is arranged in the interior of a valve chamber (26), the valve diaphragm (22) covering the valve inlet (24) with its central region.

4. Device according to any one of claims 1 to 3, **characterised in that** the valve diaphragm (22) is connected by its outer edge to the valve housing, diaphragm apertures (23) being arranged in the radial edge region of the valve diaphragm (22).

5. Device according to any one of claims 1 to 3, **characterised in that** the valve diaphragm (22) is configured as a rigid disc, which is arranged floating in the valve chamber (26).

## Revendications

1. Appareil médical sous la forme d'un cathéter (3) servant à administrer, à évacuer du liquide dans ou hors de cavités corporelles, lequel présente une tête de cathéter (1) et une tige de cathéter (2), sachant que la tête de cathéter (1) présente au niveau de l'extrémité proximale (4) une entrée (5) pourvue d'une soupape à traverser (6) assurant l'étanchéité de manière automatique et servant à introduire une canule ou une aiguille de Verres (18) dans le cathéter (3), et sachant qu'est prévu dans la zone entre la soupape à traverser (6) et la zone de la tige de cathéter (2), prévue aux fins de l'introduction dans le corps, un raccord de dérivation (8) qui est relié par l'intermédiaire d'un flexible de dérivation (9) à un ensemble de pompes d'aspiration (10), sachant que l'ensemble de pompes d'aspiration (10) est réalisé sous la forme d'une pièce en T, qui présente une soupape d'aspiration (11) dans une branche de la pièce en T menant au flexible de dérivation (9), une soupape de retenue (15) dans une branche de la pièce en T menant au raccord de la poche à sécrétion (14) ainsi qu'une branche faisant office de raccord d'injection (13), **caractérisé en ce qu'**une soupape d'arrêt (7) à actionnement manuel est disposée entre la soupape à traverser (6) de la tête de cathéter (1) et le raccord de dérivation (8).

2. Appareil selon la revendication 1, **caractérisé en ce que** la soupape d'aspiration (11) et la soupape de retenue (15) sont réalisées sous la forme de soupapes de retenue à membrane.

3. Appareil selon la revendication 1 ou 2, **caractérisé en ce qu'**une membrane de soupape (22) en forme de disque est disposée à l'intérieur d'une chambre de soupape (26), sachant que la membrane de soupape (22) recouvre l'entrée de soupape (24) par sa zone médiane.

4. Appareil selon l'une quelconque des revendications 1 à 3, **caractérisé en ce que** la membrane de soupape (22) est reliée par son bord extérieur au carter de soupape, sachant que des passages de membrane (23) sont disposés dans la zone de bord radiale de la membrane de soupape (22).

5. Appareil selon l'une quelconque des revendications 1 à 3, **caractérisé en ce que** la membrane de soupape (22) est réalisée sous la forme d'un disque rigide, laquelle est disposée de manière flottante dans la chambre de soupape (26).
